# EUROPEAN PATENT APPLICATION

(11) **EP 4 483 831 A1**
(43) Date of publication of application: **01.01.2025**
(21) Application number: 23306061.5
(22) Date of filing: 29.06.2023
(51) Int. Cl.: A61B 34/00, A61B 34/30, A61B 17/00, A61B 1/00, A61B 90/50, A61B 90/60, A61B 90/00

(54) **ROBOTIC ASSEMBLY FOR A SURGICAL SYSTEM**

(71) Applicant: Zentact Robotics, 38400 Saint Martin d'Hères (FR)
(72) Inventor: LOMBARD, Bertrand, 69100 VILLEURBANNE (FR)
(74) Representative: Regimbeau

(57) **Abstract**

The present invention concerns a robotic assembly (110) comprising:
a robotic arm (111) with at least two motorized degrees of freedom,
one medical instrument (112) coupled with the robotic arm (111),
wherein the medical instrument (112) is coupled with a flange of the robotic arm (111), this flange comprising a joint (113) configured for the transmission of at least one angular rotation and wherein the medical instrument (112) extends between the joint (113) configured for the transmission of at least one angular rotation and a pivot point (120).

## Description

The present disclosure relates to the domain of surgical systems, and particularly to surgical systems adapted to be used for ear, nose, throat (ENT) surgeries.

ENT surgeries are generally carried out as minimally invasive surgeries as all the surgical instruments needed to perform such surgeries are inserted in the patient through natural orifices.

Currently, the ENT surgeons have to hold, manually, an endoscope which allows them to see inside the cavity where they have to perform the concerned surgery and one or several surgical instrument to actually perform said surgery. Holding and actuating simultaneously as many objects renders their manipulation particularly complex and thus leads to long learning curves and multiple potential errors.

Robotized endoscopes have been developed to help surgeons perform minimally invasive surgeries, but they still remain cumbersome and very complex to manipulate. Also, most operators have difficulties to coordinate the displacements of the medical instruments with the images provided by such robotized endoscope.

For instance, document US20190374129A1 discloses an endoscope adapted to be inserted through small incision or natural orifices of a human body and equipped with tracking sensors, thus permitting to the operator to know the current pose of said endoscope within the body of the treated patient. Even if this kind of system improves the spatial coordination with respect to image capture, it requires a long learning process for the surgeon as he/she must coordinate his/her movements with what he/she sees on a display.

Document US20180214226A1 describes a robotic system which is permits to drive, tele-operatively, at least two flexible instruments, both received in a canula extending through an incision realized in the skin of the patient. This kind of system also requires a long learning process for the surgeon as tele-operating flexible instruments is a complex task.

Document WO2021/198663A1 finally describes a robotic system particularly adapted to operate laparoscopies, with three robotic arms which respectively hold one surgical instrument. This robotic system is well adapted to perform said laparoscopies wherein several incisions can be realized in the patient's skin. Such kind of robotic however is not adapted to perform ENT (ear, nose, throat) surgeries which require to have at least two, often three, distinct instruments passing through a small natural channel, such as the ear canal for instance. The robotic system described in the document WO2021/198663A1 is indeed way too cumbersome to be used for this kind of surgeries.

Finally, all the systems disclosed above aim at stabilizing movements of the endoscope but fail to provide stability to the surgeon's hands holding the surgical instruments used to actually perform the concerned surgery.

Thus, there remains a need to develop a new kind of robot with a chosen and stable point of view which permits the surgeon to, simultaneously, see the interior of the concerned cavity, for instance while have both his/her hands free to manipulate one or several surgical instruments.

An objective of the present disclosure is to provide a compact robotic assembly for holding a surgical instrument, such as an endoscope which aims at solving at least the above-mentioned drawbacks.

An object of the present invention thus concerns a robotic assembly comprising:
a. a robotic arm with at least two motorized degrees of freedom,
b. one medical instrument coupled with the robotic arm,
wherein the medical instrument is coupled with a flange of the robotic arm, this flange comprising a joint configured for the transmission of at least one angular rotation and wherein the medical instrument extends between the joint and a pivot point.

The robotic assembly can be completed by one or several of the following features, taken alone or in combination.

The medical instrument extends, at least between the flange and the pivot point, along a main axis of extension and the at least two motorized degrees of freedom of the robotic arm are configured to permit displacement of the medical instrument with respect to the pivot point according to one translational degree of freedom along its main axis of extension and according to one rotational degree of freedom around its main axis of extension.

The robotic arm comprises at least four motorized degrees of freedom. According to this aspect, the robotic arm is configured to displace the medical instrument according to at least four degrees of freedom.

The joint of the flange by which the medical instrument is coupled to the robotic arm is a passive joint. By "passive joint", we here refer to a joint deprived of motor. Thus, the robotic arm presents at least four motorized joints and one passive joint.

A first distance measured between the joint and the pivot point is at least three times greater than a second distance measured between the pivot point and a tip of the medical instrument. Advantageously, the first distance is at least four times greater than the second distance.

The medical instrument is an endoscope.

The robotic arm is linked to the pivot point by a link providing at least two degrees of freedom to the pivot point. The two degrees of freedom of the pivot point can be motorized degrees of freedom or passive degrees of freedom.

The present disclosure further concerns a surgical system comprising at least one robotic assembly as described above and at least one armrest rigidly linked to the robotic assembly.

The surgical system can be completed by one or several of the following features taken alone or in combination.

The surgical system comprises two armrests distributed on both sides of the medical instrument. Each armrest comprises at least a first part and a second part linked to one another by a passive lockable joint.

The surgical system comprises a transversal support supporting the robotic arm of the robotic assembly and the armrest(s). The transversal support presents a length, measured along a straight-line extending between fixation points of each armrests on the transversal support, smaller than 70 cm. Advantageously, the transversal support presents a length smaller than 50 cm. Optionally, the length of the transversal support can be adjustable. For instance, the transversal support can be telescopic.

The surgical system comprises at least one imaging device configured to capture, continuously, images of a head of an operator of the surgical system, and at least one data processor, the surgical system being operable according to a head-controlled mode, wherein the data processor is configured to, as long as the head-controlled mode is enabled:
a. detect at least two successive poses of a face or two successive poses of the eyes of the operator of the system captured by the imaging device,
b. determine a movement of the face or a movement of the eyes of the operator based on the concerned detected successive poses,
c. determine a commanded displacement of the medical instrument based on the determined movement of the face or of the eyes of the operator,
d. compute and send at least one instruction to the robotic assembly to displace the medical instrument based on the determined commanded displacement.

The endoscope is configured to displace automatically so as for a tip of a surgical tool manually manipulated by the operator to remain within a defined zone of a field of view of the endoscope. The defined zone of the field of view of the endoscope represents up to 50% of this field of view.

The surgical system comprises a trigger mechanism configured to permit the operator of the surgical system to switch between the head-controlled mode and the tool tracking mode. The trigger mechanism can be formed as a pedal. Alternatively, the trigger mechanism can be formed as a switch or a button.

The surgical system comprises a display device configured to display, at least, images taken by the endoscope. The medical instrument is a vision angulated endoscope comprising at least one encoder configured to measure an angle of rotation of the vision angulated endoscope, the data processor being configured to rotate the displayed image according to the same angle of rotation. As a result, it is possible to keep the display of the main orientation of the anatomic features stable, despite a change of point of view of the endoscope.

Other features, goals, and advantages of the present invention will appear more clearly upon reading of the detailed description that follows and with references to drawings provided by way of non-limiting examples, wherein:
- Figure 1 illustrates, schematically, a robotic assembly according to a first embodiment;
- Figure 2 illustrates, schematically, the robotic assembly according to a second embodiment;
- Figure 3 is a partial schematic view of a variant of the robotic assembly illustrated on figures 1 and 2, focused on a medical instrument held by a robotic arm of said robotic assembly;
- Figure 4 illustrates, partially, a surgical system comprising the robotic assembly of figure 2;
- Figure 5 is a representation of the surgical system used by an operator;
- Figure 6 illustrates, in a diagram, two modes of control of the surgical system illustrated on figure 5.

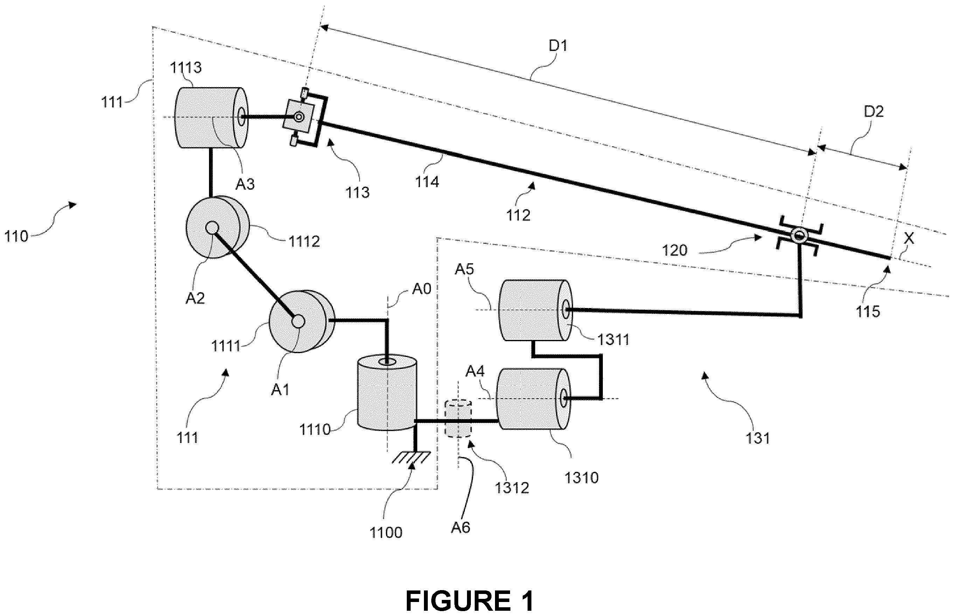

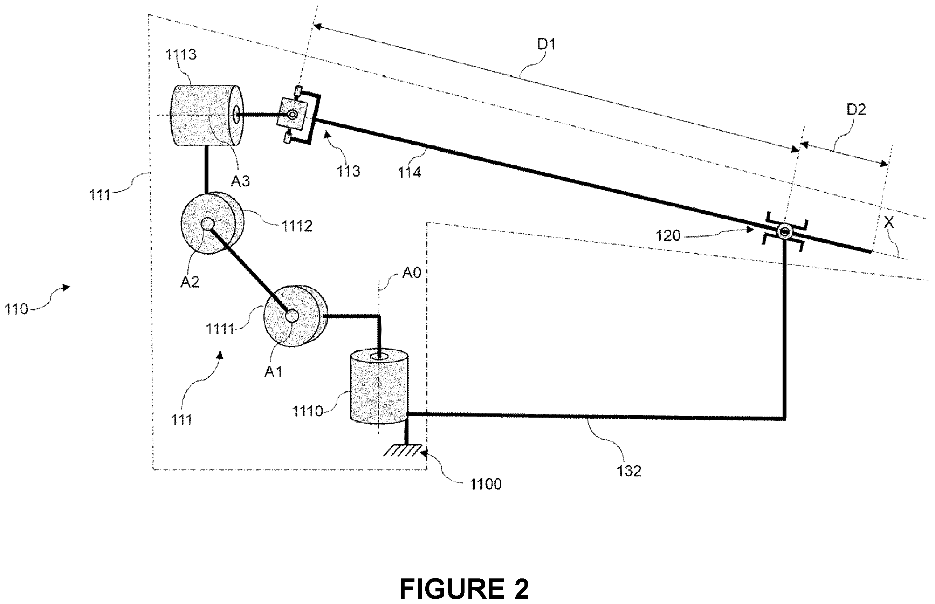

Figures 1 and 2 illustrate, schematically, a robotic assembly 110 according to, respectively, a first and a second embodiments. As shown, such robotic assembly comprises, at least a robotic arm 111 with at least two motorized degrees of freedom and one medical instrument 112 coupled with the robotic arm 111. Particularly, the robotic arm 111 is configured to permit the displacement of the medical instrument 112 according to, at least, one translational degree of freedom and one rotational degree of freedom. As detailed below, the medical instrument 112 extends, at least between the robotic arm 111 and a pivot point 120, along a main axis of extension X and the robotic arm is configured to displace such medical instrument along this main axis of extension X and around said main axis of extension X.

The illustrated robotic assembly 110 especially comprises four motorized degrees of freedom. The medical instrument 112 is coupled with a flange of the robotic arm 111. According to the illustrated embodiment, this flange comprises a joint 113 configured for the transmission of at least one angular rotation. For instance, this joint 113 can be a passive joint. By "passive joint", we here mean that said joint is deprived of motor. Thus, the illustrated robotic arm 111 comprises at least four motorized joints providing the four motorized degrees of freedom of said robotic arm 111 and one non-motorized joint 113.

Especially, a first motorized joint 1110 is a revolute joint around a first axis A0, a second motorized joint 1111 is a revolute joint around a second axis A1, a third motorized joint 1112 is a revolute joint around a third axis A2, and a fourth motorized joint 1113 is a revolute joint around a fourth axis A3. According to the illustrated embodiments, the first axis A0 is perpendicular to the second, the third and the fourth axes A1, A2, A3, the second axis A1 is parallel to the third axis A2 and the fourth axis A3 is perpendicular to the second and the third axis A0, A1, A2.

According to the present disclosure, the medical instrument 112 is an elongated medical instrument, thus comprising an elongated shaft 114 extending along the main axis X, between the joint 113 forming the flange of the robotic arm 111 and the pivot point 120, and an effective portion 115 extending beyond the pivot point 120. As mentioned above, the medical instrument 112 can be displaced according to at least two degrees of freedom with respect to this pivot point 120, namely at least one translational degree of freedom and one rotational degree of freedom, thanks to the robotic arm 111. Especially, the robotic arm of the illustrated embodiment is configured to displace the medical instrument according to at least four degrees of freedom. By way of example, the medical instrument can be an endoscope, the effective portion thus comprising an imaging apparatus such as a camera.

According to the illustrated embodiment, the joint 113 formed between the robotic arm 111 and the medical instrument 112 is more particularly formed between the fourth motorized joint 1113 and the medical instrument 112 and said joint 113 is a universal joint. As mentioned above, this joint 113 could be any kind of joint permitting to transmit an angular rotation, such as a universal joint, a beam joint, a helical joint, diaphragm joint, a flexible or elastomer joint, a jaw or lovejoy joint, a tendon joint, or a boge joint. Otherwise said, a rotation of the fourth joint 1113 around the fourth axis A3 leads to a rotation of the medical instrument 112 around its main axis of extension X.

According to an aspect of the present disclosure, a first distance D1 measured between the joint 113 forming the flange of the robotic arm 111 and the pivot point 120 is greater than a second distance D2 measured between the pivot point 120 and a tip of the medical instrument 112. For instance, the first distance D1 is at least three times greater than the second distance D2. Using such a ratio improves the accuracy when displacing the medical instrument 112. Advantageously, the first distance D1 is at least four times greater than the second distance D2.

Finally, according to the illustrated embodiments, the robotic assembly 110 comprises a link 131, 132, joining the robotic arm 111 to the pivot point 120. This link 131, 132 is configured to provide at least two degrees of freedom to the pivot point 120. These at least two degrees of freedom can be motorized degrees of freedom or passive degrees of freedom, i.e., non-motorized degrees of freedom.

According to the embodiment illustrated on figure 1, the robotic arm 111 is linked to the pivot point 120 by an articulated link 131. According to the embodiment shown, this articulated link 131 particularly comprises a first motorized joint 1310 which is a revolute joint configured to rotate around a fifth axis A4, a second motorized joint 1311 which is a revolute joint configured to rotate around a sixth axis A5 parallel to the fifth axis A4. Advantageously, this configuration permits to obtain a pivot point displaceable according to two motorized degrees of freedom. Optionally, the articulated link 131 could comprise a third motorized joint 1312 arranged before the first and the second motorized joints 1310, 1311 of this articulated link 131 and which would be a revolute joint configured to rotate around a seventh axis A6 secant, advantageously perpendicular, to the fifth and sixth axes A4, A5. As this third motorized joint 1312 is optional, it is illustrated with dotted lines. Obviously, this is only an example and the fifth and sixth axes A4, A5 could be secant within the scope of the present disclosure.

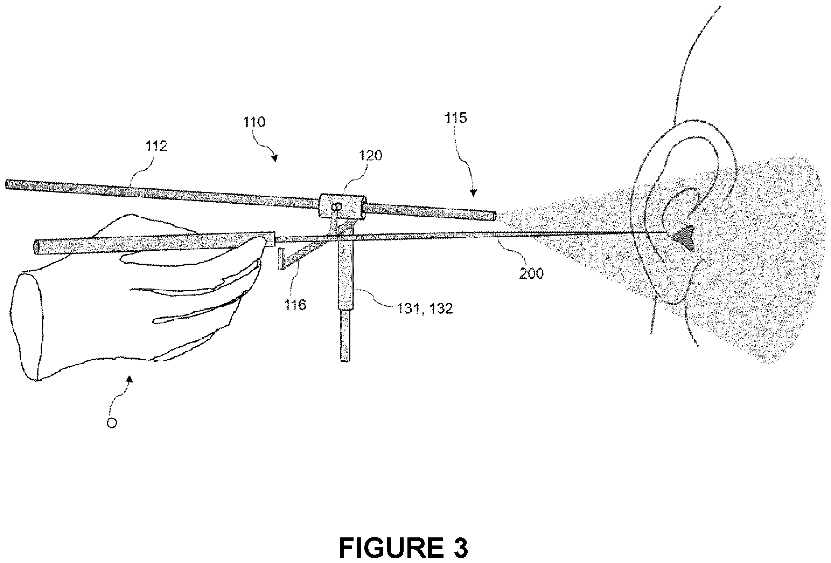

According to the second embodiment illustrated on figure 2, the robotic arm 111 can be linked to the pivot point 120 by a rigid link 132 configured to provide at least two free passive degrees of freedom to the pivot point 120. For instance, the pivot point 120 could be mounted on a universal passive joint, itself attached to the rigid link 132, as for instance partially illustrated on figure 3.

Obviously, some features of the first and the second embodiments could be combined without departing from the scope of the invention. For instance, the articulated link 131 could further be configured to provide two additional passive degrees of freedom.

According to yet another embodiment, the pivot point could be fixed in position with respect to the robotic arm.

According to a non-illustrated variant of this second embodiment, the rigid link could itself be mounted on a robotic device, for instance on a Selective Compliance Assembly Robot Arm (SCARA), thus providing, at least, three degrees of freedom which can optionally be motorized and configured to be actuated so as to follow tracked movements of the patient. For instance, the movements of the patient could be tracked thanks to an optical tracking system, a mechanical tracking system or an electromagnetic tracking system as known by the skilled man of the art. Obviously, any other known tracking system could be used with the described system. Advantageously, the joints of such a SCARA are lockable.

Obviously, these are only examples of how to carry the invention, but do not limit said invention and other configurations providing robotic arms with at least two, advantageously four, motorized degrees of freedom could be implemented without departing from the scope of the present invention.

Regardless of the embodiment implemented, the robotic assembly 110 can optionally be attached to a base 1100 arranged in a fixed position with respect to a patient support. By "patient support", we here refer to a chair or a surgical table for instance. Advantageously, the patient can be attached to the chair or to the surgical table, for instance thanks to an adhesive element, such as tape, or thanks to screws, so that the base 1100 of the surgical system 100 is in a fixed position with respect to said patient, and consequently with respect to the anatomical structure to be treated.

Figure 3 illustrates, partially, a variant of the robotic assembly 110 which comprises a support 116 configured to support and help stabilize a surgical tool 200 while said surgical tool is being held by an operator O - only a hand of said operator O being illustrated. As shown, the support 116 is particularly adapted to support an elongated surgical tool 200. The surgical tool 200 can comprise a cutting instrument, surgical forceps or canula for instance. The support 116 is attached to the articulated link 131 or to the rigid link 132, depending on which embodiment is implemented together with such variant. Especially, the illustrated pivot point 120 is here mounted on a universal joint itself mounted on the support 116.

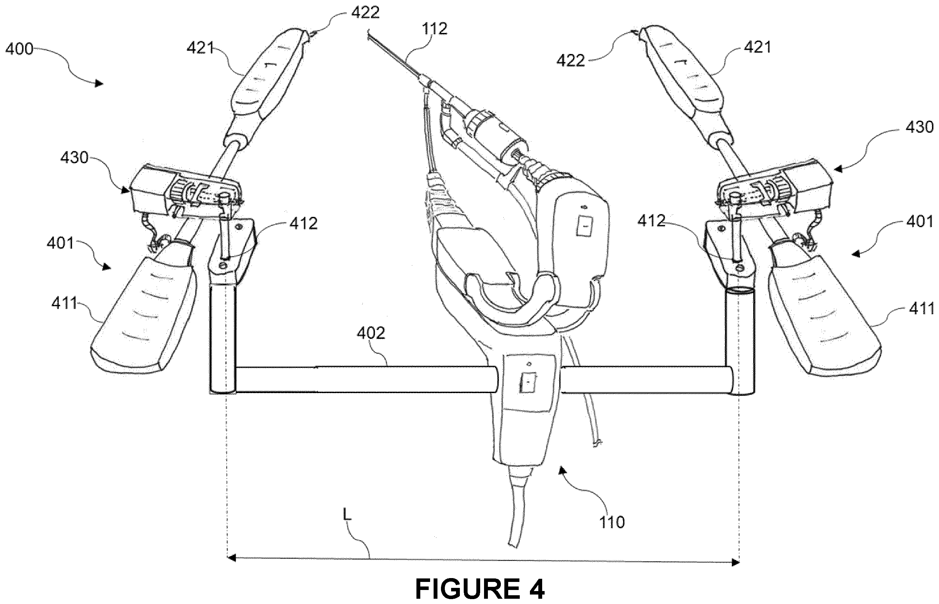

Figure 4 illustrates, schematically, a surgical system 400, comprising at least one robotic assembly 110 as described above, and at least one armrest 401 rigidly linked to the robotic assembly 110. Advantageously, the surgical system 400 comprises two armrests 401. Said armrest(s) allow(s) the operator of the surgical system 400 to stabilize his/her arms while holding surgical tools, such as cutting instruments, canula or surgical forceps for instance, thus providing a higher level of accuracy for performing the corresponding surgical act.

As shown, the armrests 401 can for instance be distributed on both sides of the robotic assembly 110, and thus on both sides of the medical instrument 112. A transversal support 402 supports, simultaneously, the robotic assembly 110 and the armrest(s) 401. Each armrest 401 is fixed to the transversal support 402 at a fixation point 412. According to this particular embodiment, the transversal support 402 thus forms a rigid link between the robotic assembly 110 and the armrest(s) 401.

Each armrest is made of two parts 411, 421 articulated with respect to each other and arranged so that a forearm of the operator can rest on a first part 411 of the armrest, while his/her hand is supported by a second part 421 of the concerned armrest. According to the illustrated embodiment, a passive lockable joint 430 links the two parts 411, 421. As schematically illustrated, each armrest comprises a switch 422 arranged on the second part 421 and configured to lock and unlock the passive lockable joint 430. When the passive lockable 430 joint is locked, the first part 411 of the concerned armrest 401 is fixed in position with respect to the second part 421 and when the passive lockable joint 430 is unlocked, the second part 421 can be freely displaced with respect to the first part 411 of said concerned armrest 401. Optionally, the passive lockable joint 430 can present an intermediate position allowing the first part 411 to be displaced with respect to the second part 421 of each armrest 401, but provided that the force applied on one of these parts is above a predetermined threshold.

Obviously, any known design can be implemented for realizing such a passive lockable joint 430.

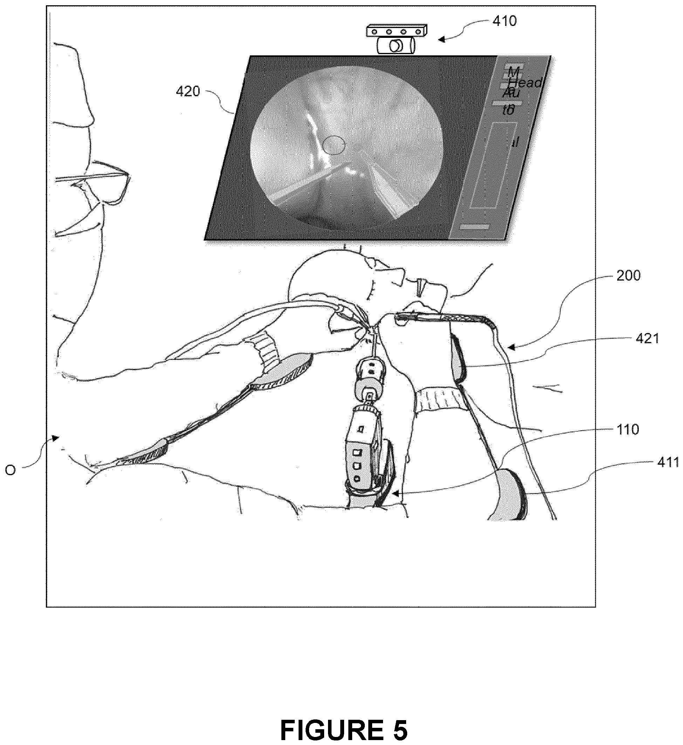

According to an aspect of the present disclosure, the transversal support 402 presents length smaller than 70 cm. Advantageously, the transversal support 402 presents a length smaller than 50 cm. By "length", we here refer to a dimension of the transversal support measured along a straight-line L extending between the fixation points 412 of each armrest on the transversal support 402. Providing a transversal support smaller than 70 cm, ensures that the whole surgical system 400 fits between the arms of the operator O of the system, as for instance illustrated on figure 5. Thus, the surgical system is not cumbersome and can be easily manipulated by said operator. Optionally, the transversal support can be adjustable, so as to permit to be adapted to any operator, for instance by providing a telescopic transversal support.

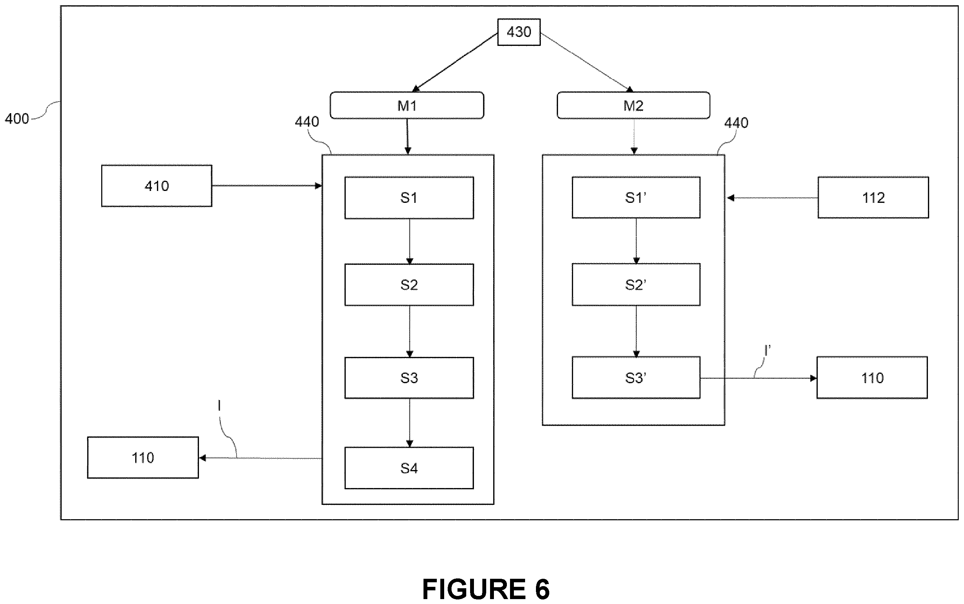

As shown on figure 5, the surgical system 400 can further comprise at least one imaging device 410 configured to capture, continuously, images of a head of the operator of the surgical system 400, and at least one data processor - schematically illustrated on figure 6 - configured to exchange data with, at least, the imaging device 410. As shown, the imaging device 410 can be mounted on a display device 420 which is configured to display images captured by the medical instrument, here formed as an endoscope. As detailed below with reference to figure 6, the imaging device 410 allows the operator of the system to control said medical instrument.

According to a particular embodiment, the endoscope can be a vision angulated endoscope. According to this particular embodiment, the vision angulated endoscope further comprises an encoder configured to measure an angle of rotation of the endoscope so that the displayed image can be rotated of the same angle of rotation, thus facilitating the interpretation of the displayed image as it permits to keep the display of the main orientation of the anatomic features stable, despite a change of point of view of the endoscope.

Figure 6 illustrates, diagrammatically, two modes of control of the surgical system 400.

As schematically illustrated, the surgical system 400 comprises a trigger mechanism 430 which allows the operator to choose between the two modes. For instance, the trigger mechanism 430 can be formed as a pedal, can be a voice-controlled command, or a button for instance arranged on one of the armrests. Any other known kind of trigger mechanism can obviously be implemented within the scope of the present invention.

The surgical system 400 can thus be operated according to a head-controlled mode M1. As illustrated, the data processor 440 is configured to, as long as the head-controlled mode is enabled:
∘ detect (step s1) at least two successive poses of a face or two successive poses of the eyes of the operator of the system captured by the imaging device 410,
∘ determine (step s2) a movement of the face or a movement of the eyes of the operator based on the detected successive poses,
∘ determine (step s3) a commanded displacement of the medical instrument based on the determined movement of the face or of the eyes of the operator,
∘ compute and send (step s4) at least one instruction i to the robotic assembly 110 to displace the medical instrument based on the determined commanded displacement.

The head-controlled mode can also be referred to as a gaze-controlled mode.

Advantageously, the imaging device 410 can be associated with an illumination, such as a LED bar - not illustrated on the figures - to improve the quality of the images taken of the operator by the imaging device. Such an illumination is configured to ensure that the operator's head is lit, particularly in an operating room where the available lights are mainly directed to the surgical site, while ensuring to not blind the operator.

Optionally, the head-controlled mode can be activated only if commanded by the operator. For instance, the surgical system 400 can comprise an actuator, such as a pedal or a button arranged on the armrest, different from the trigger mechanism, which needs to be actuated to trigger for the imaging device to start capturing images of the head of the operator. Alternatively, some movements can be locked and activated only when the actuator is actuated, while other movements can be unlocked, i.e. continuously used. Particularly, when the medical instrument is an endoscope, axial translation is often used to advance and pull back the endoscope during the whole duration of the surgery. Then, it would be uselessly complicated to subject the control of such axial translation to any actuator. Rotational movement around its main axis of extension X can also be unlocked as they are safe due the intrinsic shape of an endoscope.

Furthermore, regardless of the mode of control implemented, some safety features can be provided. For instance, the data processor can be configured to limit a maximal depth at which the endoscope can be inserted. The maximal depth can be selected by the operator of the system, or it can be preset during the planning depending on the kind of surgery to be performed for instance.

Additionally, the data processor can be configured to ensure that the medical instrument 112 remains within defined anatomical structures boundaries, such as limited by the tympanic membrane or the walls defining the ear canal for instance.

Additionally, the surgical system 400 can be operated according to a tool tracking mode M2, the data processor 440 being configured to, as long as the tool-tracking mode is enabled:
∘ identify (step 1') a current pose of the surgical tool within a field of view of the endoscope 112,
∘ determine (step 2') if the surgical tool is within a defined zone of the field of view of the endoscope 112,
∘ compute and send (step 3'), when it is determined that the surgical tool is out of the defined zone, at least one instruction I' to the motorized joints of the robotic assembly 110 to displace the endoscope so as for the surgical tool to remain within the defined zone of the field of view of the endoscope.

For instance, the defined zone of the endoscope field of view can represent up to 50% of said field of view.

The step of identifying the current pose of the surgical tool can be implemented according to different embodiments. For instance, the surgical tool can be equipped with an optical marker, for instance made as a spot of surgical paint, that the data processor 440 is able to detect. Based on the pose of this optical marker and on a known geometry of the concerned surgical tool, the data processor can determine the relative pose of such surgical tool within the field of view of the endoscope.

Alternatively, the data processor can be configured to recognize, in the images captured by the endoscope, the shape of the surgical tool, based on known technics of image segmentation. Based on this recognition, the data processor is then configured to determine the relative pose of the surgical tool within the field of view of the endoscope.

Obviously, other modes or a combination of the described modes of control of the surgical system could be implemented without departing from the scope of the invention.

## Claims

1. A robotic assembly (110) comprising:
a. a robotic arm (111) with at least two motorized degrees of freedom, and
b. one medical instrument (112) coupled with the robotic arm (111),
wherein the medical instrument (112) is coupled with a flange of the robotic arm (111), said flange comprising a joint (113) configured for transmitting at least one angular rotation to the medical instrument and wherein the medical instrument (112) extends between a pivot point (120) and the joint (113).

2. The robotic assembly (110) according to the preceding claim, wherein the medical instrument (112) extends, at least between the flange and the pivot point (120), along a main axis of extension (X) and wherein the at least two motorized degrees of freedom of the robotic arm (111) are configured to permit displacement of the medical instrument (112) with respect to the pivot point (120) according to at least one translational degree of freedom along its main axis of extension (X) and according to one rotational degree of freedom around its main axis of extension (X).

3. The robotic assembly (110) according to any of the preceding claims, wherein the robotic arm (111) comprises at least four motorized degrees of freedom.

4. The robotic assembly (110) according to any of the preceding claims, wherein the joint (113) is a passive joint.

5. The robotic assembly (110) according to any of the preceding claims, wherein a first distance (D1) measured between the joint (113) and the pivot point (120) is at least three times greater than a second distance (D2) measured between the pivot point (120) and a tip of the medical instrument (112).

6. The robotic assembly according to any of the preceding claims, wherein the medical instrument (112) is an endoscope.

7. The robotic assembly according to any of the preceding claims, wherein the robotic arm (111) is linked to the pivot point (120) by a link providing at least two degrees of freedom to the pivot point (120).

8. A surgical system (400), comprising at least one robotic assembly (110) according to any of the preceding claims, and at least one armrest (401) rigidly linked to the robotic assembly (110).

9. The surgical system (400) according to the preceding claim, comprising two armrests (401) distributed on both sides of the medical instrument (112).

10. The surgical system according to any of claims 8 or 9, comprising a transversal support (402) supporting the robotic arm (111) of the robotic assembly (110) and the armrest(s) (401).

11. The surgical system according to claim 10 in combination with claim 9, wherein the transversal support (402) presents a length, measured along a straight-line (L) extending between fixation points (412) of each armrest (401) on the transversal support (402), smaller than 70 cm.

12. The surgical system (400) according to any of claims 8 to 11, wherein each armrest (401) comprises at least a first part (411) and a second part (421) linked to one another by a passive lockable joint (430).

13. The surgical system (400) according to any of claims 8 to 12, comprising at least one imaging device (410) configured to capture, continuously, images of a head of an operator (O) of the surgical system (400), and at least one data processor (440), the surgical system (400) being operable according to a head-controlled mode, wherein the at least one data processor (440) is configured to, as long as the head-controlled mode is enabled:
a. detect at least two successive poses of a face or two successive poses of the eyes of the operator (O) of the system captured by the imaging device (410),
b. determine a movement of the face or a movement of the eyes of the operator (O) based on said detected successive poses,
c. determine a commanded displacement of the medical instrument (112) based on the determined movement of the face or of the eyes of the operator (O),
d. compute and send at least one instruction to the robotic assembly (110) to displace the medical instrument (112) based on the determined commanded displacement.

14. The surgical system (400) according to any of claims 8 to 13, wherein the medical instrument (112) is an endoscope, and wherein the data processor is configured to instruct the robotic assembly to move the endoscope so as for a tip of a surgical tool (200) manually manipulated by the operator (O) to remain within a defined zone of a field of view of the endoscope.

15. The surgical system (400) according to the preceding claim, wherein the defined zone of the field of view of the endoscope represents up to 50% of said field of view.

16. The surgical system (400) according to any of claims 14 or 15, wherein the surgical system (400) comprises a display device (420) configured to display, at least, images captured by the endoscope.

17. The surgical instrument according to the preceding claim, wherein the medical instrument (112) is a vision angulated endoscope comprising at least one encoder configured to measure an angle of rotation of the vision angulated endoscope, and wherein the data processor is configured to rotate the image displayed according to the same angle of rotation.
